# EUROPEAN PATENT SPECIFICATION

(11) **EP 3 964 156 B1**
(45) Date of publication and mention of the grant of the patent: **11.09.2024**
(21) Application number: 19927259.2
(22) Date of filing: 24.07.2019
(51) Int. Cl.: A61B 18/26

(54) **SHEATH CAP AND ENDOSCOPE INTRODUCER SHEATH WITH SHEATH CAP**
HÜLSENKAPPE UND ENDOSKOPEINFÜHRHÜLSE MIT HÜLSENKAPPE
CAPUCHON À MANCHON ET MANCHON D'INTRODUCTION D'ENDOSCOPE AVEC CAPUCHON À MANCHON

(30) Priority: 30.04.2019 CN 201910363388; 30.04.2019 CN 201920624832 U
(43) Date of publication of application: 09.03.2022
(73) Proprietor: Wuhan Youcare Technology Co., Ltd., Wuhan, Hubei 430223 (CN)
(72) Inventor: WANG, Shaogang, Wuhan, Hubei 430223 (CN); HU, Xuecheng, Wuhan, Hubei 430223 (CN); MAO, Yeyun, Wuhan, Hubei 430223 (CN); YU, Xiao, Wuhan, Hubei 430223 (CN); GUAN, Wei, Wuhan, Hubei 430223 (CN); QIN, Cao, Wuhan, Hubei 430223 (CN); LONG, Gang, Wuhan, Hubei 430223 (CN); LI, Ying, Wuhan, Hubei 430223 (CN); LIU, Chengpeng, Wuhan, Hubei 430223 (CN); LI, Wenhua, Wuhan, Hubei 430223 (CN)
(74) Representative: Zeuner Summerer Stütz
(86) International application number: PCT/CN2019/097446
(87) International publication number: WO 2020/220485

(56) References cited:
- WO-A1-2017/136347
- CN-A- 103 623 946
- CN-A- 107 898 486
- CN-A- 107 898 486
- CN-A- 108 601 622
- CN-A- 108 685 615
- CN-A- 109 316 158
- CN-A- 109 998 672
- CN-U- 202 788 631
- CN-U- 205 459 120
- CN-U- 208 709 842
- US-A1- 2017 128 083

## Description

### FIELD

The invention relates to the technical field of medical devices for human diseased tissues, and in particular to a sheath cap used for calculus in a human body cavity and an endoscope introducer sheath with the sheath cap.

### BACKGROUND

At present, the surgical treatment of diseased tissues in the human body cavity has changed from traditional open surgery to minimally invasive surgery. The main method of minimally invasive surgery to treat calculi is to establish a channel, observe in real time under an endoscope, and perform lithotripsy and stone extraction operations with a holmium laser. The commonly used treatment device is an endoscope introducer sheath, and the endoscope introducer sheath introduces the holmium laser into human body through natural orifices to pulverize stones. For some stones that cannot be powdered, a stone basket may enter a kidney from an instrument passage to take granular stones out of the body. In the lithotripsy operation, it is necessary to inject water into the stone via a water spray joint of the endoscope introducer sheath, so that the broken calculi can be separated from the calculus point after being washed by the water, and thus the stone basket can be used to enter the instrument passage to take the broken calculi out or the broken calculi can be naturally expelled from body channels. However, due to the close connection between the broken calculi and the calculus point, the broken calculi are difficult to be expelled after being pulverized by the holmium laser, and are still attached to the calculus point, which affects the effect of the operation and also increases the pain of the patient. WO 2017/136347A1 and CN107898486A disclose endoscopic sheath caps of the prior art.

### SUMMARY

Claim 1 defines the invention and dependant claims disclose embodiments. An object of the present invention is to overcome the above-mentioned prior art problems and provide a sheath cap which has a simple structure and can make a water flow effectively flush a calculus point to successfully expel broken calculi, and also an endoscope introducer sheath with the sheath cap.

To achieve the above object, a sheath cap of the present invention comprises a housing collar and a cylinder which is coaxially fixed on an inner surface of one end of the housing collar. The cylinder is internally provided with instrument channel straight holes and a water spray channel straight hole which are parallel to an axial direction of the cylinder. The sheath cap is provided with a water spray channel oblique hole, which is in communication with the water spray channel straight hole and can spray water to the lateral front side of the sheath cap.

Based on this, a the water spray channel oblique hole can be designed into the following two structural forms:
Structural form 1: the water spray channel oblique hole comprises water spray channel inclined straight holes, each of which has one end communicated with the water spray channel straight hole and the other end extending to an outer surface of the housing collar on the side close to the front end face of the cylinder,. The water spray channel inclined straight holes are arranged along a circle of the sheath cap in the circumferential direction at intervals.

In an embodiment, the angle between the water spray channel inclined straight hole and the water spray channel straight hole is 5° ~ 85°.

Structural form 2: the water spray channel oblique hole of the invention comprises water spray channel oblique slots, each of which is provided on the surface of the cylinder and forms an angle with the axial direction of the cylinder. A water spray connection channel is provided in the cylinder and is connected between the rear end of the water spray channel oblique slot and the water spray channel straight hole. The water spray channel oblique slots are provided on a surface of a circle of the cylinder in the circumferential direction at intervals.

In accordance with the present invention, the cylinder is coaxially fixed with a cylinder collar which makes a water flow be sprayed to the lateral front side of the sheath cap along the orientation of the water spray channel oblique slot, and the water outlet end of the water spray channel oblique slot is located in front of the cylinder collar.

In accordance with the present invention, the housing collar and the cylinder collar are coaxially and fixedly connected as a whole, and the outer diameter of the cylinder collar is the same as the outer diameter of the housing collar.

An endoscope introducer sheath with a sheath cap of the present invention, comprises an introducer sheath handle and an introducer sheath tube coaxially fixed on the front end of the introducer sheath handle, the introducer sheath tube is internally provided with an axial channel, the introducer sheath handle is provided with instrument joints and a water spray joint, the instrument joints and the water spray joint are respectively provided along their respective axial directions with instrument joint channels and a water spray joint channel which are in communication with the axial channel of the introducer sheath tube. An inner surface of the other end of the housing collar is coaxially fixed on the front end of the introducer sheath tube, the instrument channel straight holes and the water spray channel straight hole are in communication with the axial channel of the introducer sheath tube.

In an embodiment of the present invention, the introducer sheath tube comprises an introducer sheath rigid tube coaxially fixed on the front end of the introducer sheath handle and an introducer sheath flexible tube coaxially fixed on the front end of the introducer sheath rigid tube, and the other end of the housing collar is coaxially and fixedly connected onto the front end surface of the introducer sheath flexible tube, the housing collar is fixedly connected with a pull wire, and the pull wire passes through the axial channel of the introducer sheath tube to be connected with a flexible tube bending control device, and the flexible tube bending control device is provided on the introducer sheath handle.

In another embodiment of the present invention, the instrument joints comprise a holmium laser joint and an image illumination device joint, the holmium laser joint and the image illumination device joint are respectively provided along their respective axial directions with a holmium laser joint channel and an image illumination device joint channel which are in communication with the axial channel of the introducer sheath tube, the instrument channel straight holes comprise a holmium laser channel straight hole and an image illumination device channel straight hole. The holmium laser channel straight hole, the image illumination device channel straight hole and the water spray channel straight hole are in communication with the axial channel of the introducer sheath tube.

In still another embodiment of the present invention, an axial partition is fixed in the introducer sheath tube, and the axial partition divides the axial channel of the introducer sheath tube into a first passage communicated with the holmium laser joint channel and a second passage communicated with the image illumination device joint channel and the water spray joint channel. The aperture of the second passage is larger than the aperture of the first passage. The first passage is coaxially communicated with the holmium laser channel straight hole, and the second passage is communicated with the image illumination device channel straight hole and the water spray channel straight hole.

The advantages associated with the present invention are as follows: by designing the form of a combined hole structure combining the water spray channel straight hole and the water spray channel oblique hole in the sheath cap, a water flow entering from the endoscope introducer sheath can be simultaneously sprayed to the front and the side of a calculus point in front of the sheath cap. While broken calculi at the calculus point are subjected to multi-angle washing to increase the probability of the broken calculi being removed from the calculus point, the front water flow and the side water flow of the sheath cap interact with each other to form a vortex, and the broken calculi is further separated from the calculus point under the influence of the vortex, thereby increasing the probability of separation between the broken calculi and the calculus point, and ensuring the washing effect of the water flow on the broken calculi. The endoscope introducer sheath is used in conjunction with the sheath cap. During the operation, the endoscope introducer sheath enters human body cavity under the introduction of the sheath tube, the image illumination device enters the front end of the endoscope introducer sheath via the image illumination device joint. The water spray joint is connected to a water pipe, and thus the water flow is sprayed out in the human body cavity through the water spray channel oblique hole and the water spray channel straight hole, flushing the visual field to ensure that the image illumination device transmits a clear image. Operate the introducer sheath handle, move the endoscope introducer sheath back and forth, and adjust the bending angle of the introducer sheath tube in the cavity by the flexible tube bending control device to find stones. After finding out the stone, holmium laser is provided via the holmium laser joint and is focused on the stone to crush it. The powdered stone is flushed out of the body through water circulation.

The sheath cap and the endoscope introducer sheath designed by the present invention can effectively treat human calculus, and are easy to operate, greatly reduce the pain of patients, and have good therapeutic effects and high market application value.

### BRIEF DESCRIPTION OF THE DRAWINGS

FIG. 1 is a first perspective view of a sheath cap;
FIG. 2 is a second perspective view of the sheath cap ;
FIG. 3 is a third perspective view of the sheath cap ;
FIG. 4 is a front view of the sheath cap ;
FIG. 5 is a top view of the sheath cap ;
FIG. 6 is a cross-sectional view of the sheath cap shown in FIG. 5 along the line A-A;
FIG. 7 is a perspective view of a sheath cap according to the present invention;
FIG. 8 is an exploded view of the sheath cap according to the present invention;
FIG. 9 is a front view of the sheath cap according to the present invention;
FIG. 10 is a cross-sectional view of the sheath cap shown in FIG. 9 along the line B-B;
FIG. 11 is a perspective view of an endoscope introducer sheath with a sheath cap according to the present invention;
FIG. 12 is a front view of the endoscope introducer sheath with a sheath cap according to the present invention;
FIG. 13 is a cross-sectional view of the endoscope introducer sheath with the sheath cap shown in FIG. 12 along the line C-C;
FIG. 14 is an enlarged view of the part marked by symbol D shown in FIG. 12;

Among them, 1 - housing collar, 2 - cylinder, 3 -water spray channel straight hole, 4 - water spray channel oblique hole ( 4.1 - water spray channel inclined straight hole, 4.2 - water spray channel oblique slot, 4.3 - water spray connection channel), 5 - cylinder collar, 6 - introducer sheath handle, 7 - introducer sheath tube (7.1 - introducer sheath rigid tube, 7.2 - introducer sheath flexible tube), 8 - water spray joint, 9 - water spray joint channel, 10 - flexible tube bending control device, 11 - holmium laser joint, 12 - image illumination device joint, 13 - holmium laser joint channel, 14 - image illumination device joint channel, 15 - holmium laser channel straight hole, 16 - image illumination device channel straight hole, 17 - axial partition, 18 - first passage, 19 - second passage, 20 - holmium laser connection tube, 21 - water inlet connection tube, 22 - image connection tube.

### DETAILED DESCRIPTIONS

The present disclosure will be further described in detail below in conjunction with the drawings and specific embodiments.

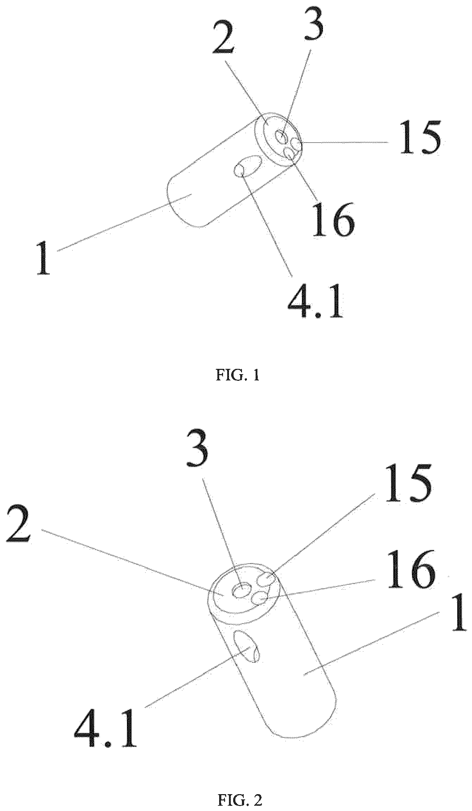

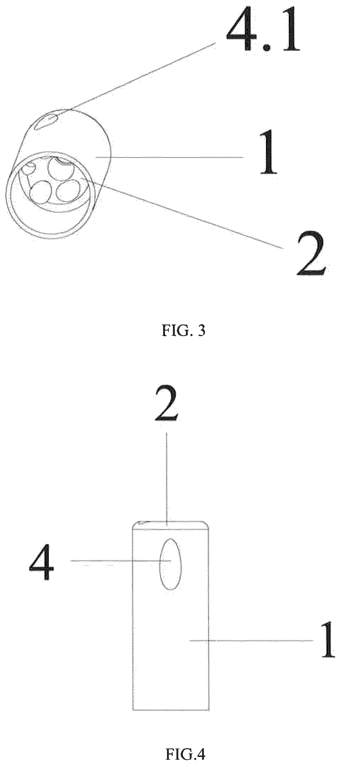

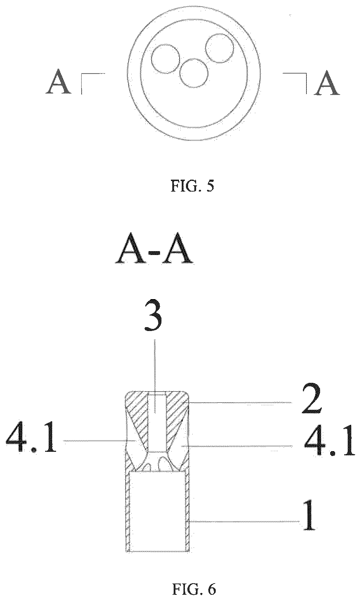

FIGs. 1-6 shows an embodiment of a sheath cap, which comprises a housing collar 1 and a cylinder 2 which is coaxially fixed on an inner surface of one end of the housing collar 1. The cylinder 2 is internally provided with an image illumination device channel straight hole 16, a holmium laser channel straight hole 15 and a water spray channel straight hole 3 which are parallel to an axial direction of the cylinder 2. The sheath cap is provided with a water spray channel oblique hole 4, which is in communication with the water spray channel straight hole 3 and can spray water to the lateral front side of the sheath cap. The water spray channel oblique hole 4 comprises water spray channel inclined straight holes 4.1, each of which has one end communicated with the water spray channel straight hole 3 and the other end extending to an front end surface of the housing collar 1. The water spray channel inclined straight holes 4.1 are symmetrically provided on the left and right sides of the sheath cap.

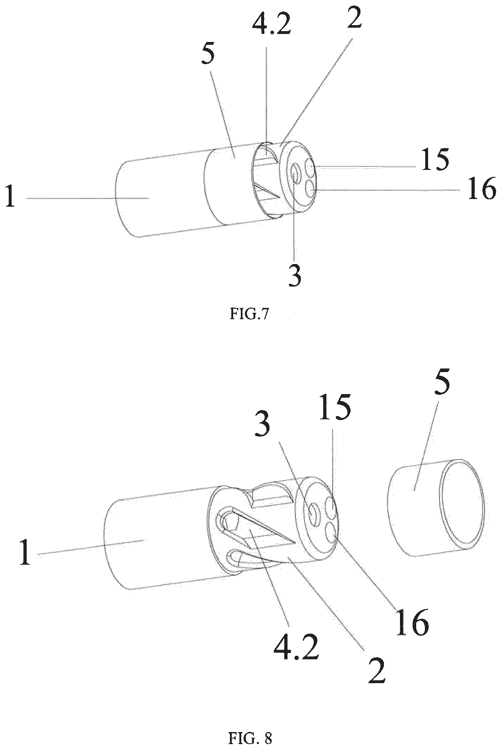

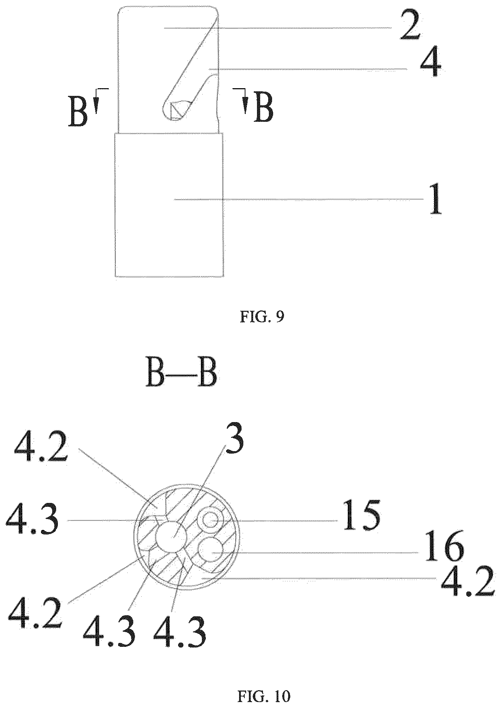

FIGs. 7 - 10 shows anembodiment of a sheath cap of the invention, which comprises a housing collar 1 and a cylinder 2 which is coaxially fixed on an inner surface of one end of the housing collar 1. The cylinder 2 is internally provided with an image illumination device channel straight hole 16, a holmium laser channel straight hole 15 and a water spray channel straight hole 3 which are parallel to an axial direction of the cylinder 2. The sheath cap is provided with a water spray channel oblique hole 4, which is in communication with the water spray channel straight hole 3 and can spray water to the lateral front side of the sheath cap. The water spray channel oblique hole 4 comprises water spray channel oblique slots 4.2, each of which is provided on the surface of the cylinder 2 and forms an angle with the axial direction of the cylinder 2. A water spray connection channel 4.3 is connected between the rear end of the water spray channel oblique slot 4.2 and the water spray channel straight hole 3 and is provided in the cylinder 2. The water spray channel oblique slots 4.2 are provided on the circumferential surface of the cylinder 2 at intervals. The cylinder 2 is coaxially fixed with a cylinder collar 5. The water outlet end of the water spray channel oblique slot 4.2 is located in front of the cylinder collar 5. The housing collar 1 and the cylinder collar 5 are coaxially and fixedly connected as a whole, and the outer diameter of the cylinder collar 5 is the same as the outer diameter of the housing collar 1.

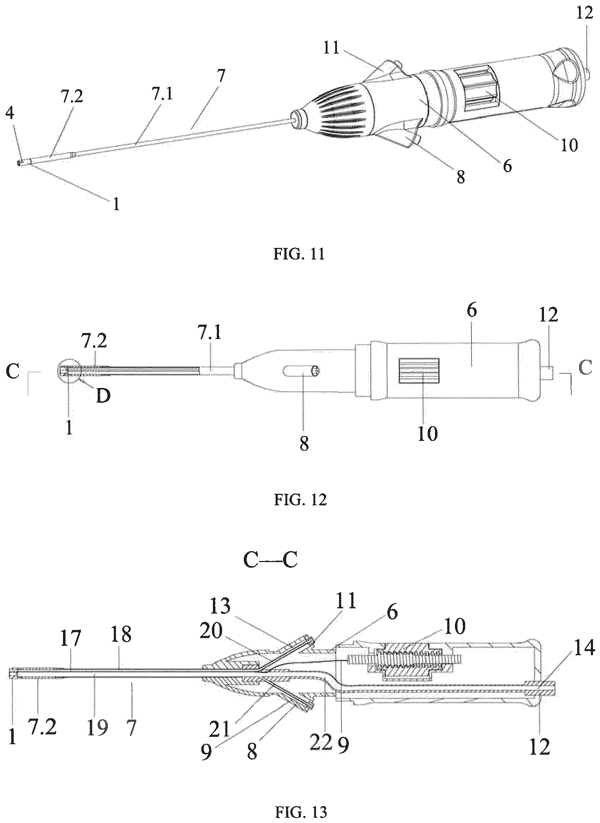

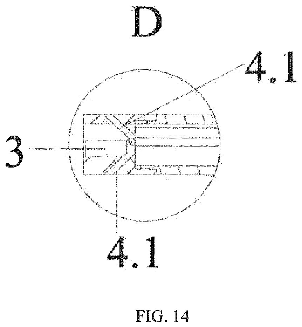

FIGs. 11 - 14 shows an embodiment of an endoscope introducer sheath with a sheath cap, which comprises an introducer sheath handle 6 and an introducer sheath tube 7 coaxially fixed on the front end of the introducer sheath handle 6. The introducer sheath handle 6 is provided with a holmium laser joint 11, an image illumination device joint 12 and a water spray joint 8. The holmium laser joint 11, the image illumination device joint 12 and the water spray joint 8 are respectively provided along their respective axial directions with a holmium laser joint channel 13 and an image illumination device joint channel 14 and a water spray joint channel 9. The holmium laser joint channel 13 and the image illumination device joint channel 14 and the water spray joint channel 9 are respectively communicated with an axial channel of the introducer sheath tube 7 through a holmium laser connection tube 20, an image connection tube 22 and a water inlet connection tube 21.

The introducer sheath tube 7 comprises an introducer sheath rigid tube 7.1 coaxially fixed on the front end of the introducer sheath handle 6 and an introducer sheath flexible tube 7.2 coaxially fixed on the front end of the introducer sheath rigid tube 7.1. The other end of the housing collar 1 is coaxially and fixedly connected onto the front end surface of the introducer sheath flexible tube 7.2. The housing collar 1 is fixedly connected with a pull wire 9, and the pull wire 9 passes through the axial channel of the introducer sheath tube 7 and is connected with a flexible tube bending control device 10. The flexible tube bending control device 10 is provided on the introducer sheath handle 6. A structure combining a thumb wheel and a thumb wheel lever could be used as the flexible tube bending control device 10 of the present invention. However, the present invention is not limited as such, other structures such as a structure combining an activation lever and a spinning reel can also be used as the flexible tube bending control device 10. By rotating the thumb wheel, the thumb wheel lever is driven to move forward and backward, thereby turning the pull wire 9 to move forward and backward in order to make the introducer sheath flexible tube 7.2 bend. An axial partition 17 is fixed in the introducer sheath tube 7, and the axial partition 17 divides the axial channel of the introducer sheath tube 7 into a first passage 18 communicated with the holmium laser joint channel 13 and a second passage 19 communicated with the image illumination device joint channel 14 and the water spray joint channel 9. The aperture of the second passage 19 is larger than the aperture of the first passage 18. The first passage 18 is coaxially communicated with the holmium laser channel straight hole 15, and the second passage 19 is communicated with the image illumination device channel straight hole 16 and the water spray channel straight hole 3.

In the present invention, based on the holmium laser channel straight hole 15, the image illumination device channel straight hole 16 and the water spray channel straight hole 3, the water spray channel oblique hole 4 is designed in the sheath cap, making the water spray channel oblique hole 4 be in communication with the water spray channel straight hole 3. The water flow can be sprayed to the front and the lateral front side of the sheath cap when passing through the sheath cap and intending to be sprayed to the front of it. The water flow in two directions effectively strikes the broken calculi, and at the same time, the water flows in two directions interact with each other to form a vortex, which helps the floating and discharging of the broken calculi, thereby the present invention has a simple structure and high practicality.

As shown in FIGs.11 - 14, an endoscope introducer sheath is used in conjunction with the sheath cap. During the operation, the endoscope introducer sheath enters human body cavity under the introduction of the sheath tube, the image illumination device enters the front end of the endoscope introducer sheath via the image illumination device joint 12, and the water spray joint 8 is connected to a water pipe, and thus water flow is sprayed out in the human body cavity through the water spray channel oblique hole 4 and the water spray channel straight hole 3, flushing the visual field to ensure that the image illumination device transmits a clear image. Operate the introducer sheath handle 6, move the introducer sheath handle 6 back and forth, and adjust the bending angle of the introducer sheath flexible tube 7.2 in the cavity by the flexible tube bending control device 10 to find the stones. After finding out the stone, the holmium laser is provided via the holmium laser joint and is focused on the stone to crush it. The powdered stone is flushed out of the body through water circulation.

In the present invention, the advantage of the endoscope introducer sheath used in conjunction with the sheath cap is that the introducer sheath tube 7 only has two passages, and there is only one wall between the passages, which can save space. The introducer sheath rigid tube 7.1 does not need to be bent , the wall thickness of introducer sheath rigid tube 7.1 can be thinner than that of the introducer sheath flexible tube 7.2, and the series of improvements enables the outer diameter of the introducer sheath rigid tube 7.1 to be much smaller (the introducer sheath flexible tube 7.2 and introducer sheath rigid tube 7.1 can have the same outer diameter to ensure the strength of the overall structure, or the introducer sheath flexible tube 7.2 can be thinner than the introducer sheath rigid tube 7.1 so that the introducer sheath flexible tube 7.2 can enter small cavities more easily, and the considerations can be selected according to the actual circumstances). During the operation, the endoscope introducer sheath carries the image illumination device, holmium laser and water flow into the kidney. Because the outer diameter of the introducer sheath rigid tube 7.1 is smaller than that of the introducer sheath flexible tube 7.2, the gap between the introducer sheath rigid tube 7.1 and the wall of the cavity is large, which is conducive for the water in the kidney to flow out quickly and thus the pressure in the kidney is lowered.

The sheath cap and the combination set of the sheath cap and the endoscope introducer sheath of the present invention can effectively treat human calculus, and are easy to operate. The present invention can greatly reduce the pain of patients, only one surgery can completely cure the stones in kidney calices and have good therapeutic effects and high market application value.

The foregoing are only preferred embodiments of the present invention and is not meant to place any formally restriction on the structure of the invention. It should be understood that various changes, substitutions and alterations made according to the nature of the technical disclosure are intended to fall within the scope of the present invention, the scope of which is defined by the claims.

## Claims

1. A sheath cap, comprising a housing collar (1) and a cylinder (2) which is coaxially fixed on an inner surface of one end of the housing collar (1), the cylinder (2) being internally provided with instrument channel straight holes and a water spray channel straight hole (3) which are parallel to an axial direction of the cylinder, wherein the sheath cap is provided with a water spray channel oblique hole (4) which is in communication with the water spray channel straight hole (3) and can spray water to the lateral front side of the sheath cap;
**characterised in that** the water spray channel oblique hole (4) comprises water spray channel oblique slots (4.2), each of which is provided on the surface of the cylinder (2) and forms an angle with the axial direction of the cylinder (2), a water spray connection channel (4.3) is provided in the cylinder (2) and is connected between the rear end of the water spray channel oblique slot (4.2) and the water spray channel straight hole (3), the water spray channel oblique slots (4.2) are provided on a surface of a circle of the cylinder (2) in the circumferential direction at intervals;
the cylinder (2) is coaxially fixed with a cylinder collar (5) which makes a water flow be sprayed to the lateral front side of the sheath cap along the orientation of the water spray channel oblique slot (4.2), and the water outlet end of the water spray channel oblique slot (4.2) is located in front of the cylinder collar (5); and the housing collar (1) and the cylinder collar (5) are coaxially and fixedly connected as whole, and the outer diameter of the cylinder collar (5) is the same as the outer diameter of the housing collar (1).

2. An endoscope introducer sheath with a sheath cap, comprising an introducer sheath handle (6) and an introducer sheath tube (7) coaxially fixed on the front end of the introducer sheath handle (6), the introducer sheath tube (7) being internally provided with an axial channel, the introducer sheath handle (6) being provided with instrument joints and a water spray joint (8), the instrument joints and the water spray joint (8) being respectively provided along their respective axial directions with instrument joint channels and a water spray joint channel (9) which are in communication with the axial channel of the introducer sheath tube (7), wherein the endoscope introducer sheath further comprises the sheath cap of claim 1, an inner surface of the other end of the housing collar (1) is coaxially fixed on the front end of the introducer sheath tube (7), the instrument channel straight holes and the water spray channel straight hole (3) are in communication with the axial channel of the introducer sheath tube (7).

3. The endoscope introducer sheath with sheath cap of claim 2,wherein the introducer sheath tube (7) comprises an introducer sheath rigid tube (7.1) coaxially fixed on the front end of the introducer sheath handle (6) and an introducer sheath flexible tube (7.2) coaxially fixed on the front end of the introducer sheath rigid tube (7.1) , and the other end of the housing collar (1) is coaxially and fixedly connected onto the front end surface of the introducer sheath flexible tube (7.2), the housing collar (1) is fixedly connected with a pull wire (9), and the pull wire (9) passes through the axial channel of the introducer sheath tube (7) to be connected with a flexible tube bending control device (10), and the flexible tube bending control device (10) is provided on the introducer sheath handle (6).

4. The endoscope introducer sheath with sheath cap of claim 3,wherein the instrument joints comprise a holmium laser joint (11) and an image illumination device joint (12), the holmium laser joint (11) and the image illumination device joint (12) are respectively provided along their respective axial directions with a holmium laser joint channel (13) and an image illumination device joint channel (14) which are in communication with the axial channel of the introducer sheath tube (7), the instrument channel straight holes comprise a holmium laser channel straight hole (15) and an image illumination device channel straight hole (16), the holmium laser channel straight hole (15), the image illumination device channel straight hole (16) and the water spray channel straight hole (3) are in communication with the axial channel of the introducer sheath tube (7).

5. The endoscope introducer sheath with sheath cap of claim 4,wherein an axial partition (17) is fixed in the introducer sheath tube (7), and the axial partition (17) divides the axial channel of the introducer sheath tube (7) into a first passage (18) communicated with the holmium laser joint channel (13) and a second passage communicated (19) with the image illumination device joint channel (14) and the water spray joint channel (9), the aperture of the second passage (19) is larger than the aperture of the first passage (18), the first passage (18) is coaxially communicated with the holmium laser channel straight hole (15), and the second passage (19) is communicated with the image illumination device channel straight hole (16) and the water spray channel straight hole (3).

## Patentansprüche

1. Hülsenkappe, umfassend einen Gehäusekragen (1) und einen Zylinder (2), der koaxial auf einer Innenoberfläche eines Endes des Gehäusekragens (1) fixiert ist, wobei der Zylinder (2) innen mit geraden Instrumentenkanallöchern und einem geraden Wassersprühkanalloch (3) ausgestattet ist, die parallel zu einer axialen Richtung des Zylinders verlaufen, wobei die Hülsenkappe mit einem schrägen Wassersprühkanalloch (4) ausgestattet ist, das in Kommunikation mit dem geraden Wassersprühkanalloch (3) ist und Wasser zu einer lateralen Vorderseite der Hülsenkappe sprühen kann;
**dadurch gekennzeichnet, dass** das schräge Wassersprühkanalloch (4) schräge Wassersprühkanalschlitze (4.2) umfasst, von denen jeder auf der Oberfläche des Zylinders (2) bereitgestellt wird und einen Winkel mit der axialen Richtung des Zylinders (2) bildet, in dem Zylinder (2) ein Wassersprühverbindungskanal (4.3) bereitgestellt wird und zwischen dem hinteren Ende des schrägen Wassersprühkanalschlitzes (4.2) und dem geraden Wassersprühkanalloch (3) verbunden ist, wobei die schrägen Wassersprühkanalschlitze (4.2) auf einer Oberfläche eines Kreises des Zylinders (2) in der Umfangrichtung in Intervallen bereitgestellt werden; der Zylinder (2) koaxial mit einem Zylinderkragen (5) fixiert ist, der bewirkt, dass ein zu der lateralen Vorderseite der Hülsenkappe zu sprühender Wasserfluss sich entlang der Orientierung des schrägen Wassersprühkanalschlitzes (4.2) bewegt, und das Wasserauslassende des schrägen Wassersprühkanalschlitzes (4.2) sich vor dem Zylinderkragen (5) befindet; und
der Gehäusekragen (1) und der Zylinderkragen (5) koaxial und fixiert als Ganzes verbunden sind, und der Außendurchmesser des Zylinderkragens (5) der gleiche wie der Außendurchmesser des Gehäusekragens (1) ist.

2. Endoskopeinführhülse mit einer Hülsenkappe, umfassend einen Einführhülsengriff (6) und ein Einführhülsenrohr (7), das koaxial an dem vorderen Ende des Einführhülsengriffes (6) fixiert ist, wobei das Einführhülsenrohr (7) innen mit einem axialen Kanal ausgestattet ist, der Einführhülsengriff (6) mit Instrumentenverbindungsstücken und einem Wassersprühverbindungsstück (8) ausgestattet ist, die Instrumentenverbindungsstücke und das Wassersprühverbindungsstück (8) jeweils entlang ihrer jeweiligen axialen Richtungen mit Instrumentenverbindungsstückkanälen und einem Wassersprühverbindungsstückkanal (9) ausgestattet sind, die in Kommunikation mit dem axialen Kanal des Endoskopeinführrohrs (7) sind, wobei die Endoskopeinführhülse des Weiteren die Hülsenkappe gemäß Anspruch 1 umfasst, eine Innenoberfläche des anderen Endes des Gehäusekragens (1) koaxial an dem vorderen Ende des Einführhülsenrohrs (7) fixiert ist, die geraden Instrumentenkanallöcher und das gerade Wassersprühkanalloch (3) in Kommunikation mit dem axialen Kanal des Einführhülsenrohrs (7) sind.

3. Endoskopeinführhülse mit Hülsenkappe nach Anspruch 2, wobei das Einführhülsenrohr (7) ein starres Einführhülsenrohr (7.1), das koaxial an dem vorderen Ende des Einführhülsengriffs (6) fixiert ist, und ein flexibles Einführhülsenrohr (7.2) umfasst, das koaxial an dem vorderen Ende des starren Einführhülsenrohrs (7.1) fixiert ist, und das andere Ende des Gehäusekragens (1) koaxial und fixiert mit der vorderen Endoberfläche des flexiblen Einführhülsenrohrs (7.2) verbunden ist, der Gehäusekragen (1) fixiert mit einem Zugdraht (9) verbunden ist, und der Zugdraht (9) den axialen Kanal des Einführhülsenrohrs (7) passiert, um mit einer Biegesteuervorrichtung (10) des flexiblen Rohrs verbunden zu werden, und die Biegesteuervorrichtung (10) des flexiblen Rohrs auf dem Einführhülsengriff (6) bereitgestellt wird.

4. Endoskopeinführhülse mit Hülsenkappe nach Anspruch 3, wobei die Instrumentenverbindungsstücke ein Holmium-Laserverbindungsstück (11) und ein Bildbeleuchtungsvorrichtungsverbindungsstück (12) umfassen, wobei das Holmium-Laserverbindungsstück (11) und das Bildbeleuchtungsvorrichtungsverbindungsstück (12) jeweils entlang ihrer jeweiligen axialen Richtungen mit einem Holmium-Laserverbindungsstückkanal (13) und einem Bildbeleuchtungsvorrichtungsverbindungsstückkanal (14) ausgestattet sind, die in Kommunikation mit dem axialen Kanal des Einführhülsenrohrs (7) sind, wobei die geraden Instrumentenkanallöcher ein gerades Holmium-Laserkanalloch (15) und ein gerades Bildbeleuchtungvorrichtungskanalloch (16) umfassen, wobei das gerade Holmium-Laserkanalloch (15), das gerade Bildbeleuchtungsvorrichtungskanalloch (16) und das gerade Wassersprühkanalloch (3) in Kommunikation mit dem axialen Kanal des Einführhülsenrohrs (7) sind.

5. Endoskopeinführhülse mit Hülsenkappe nach Anspruch 4, wobei eine axiale Unterteilung (17) in dem Einführhülsenrohr (7) fixiert ist, und die axiale Unterteilung (17) den axialen Kanal des Einführhülsenrohrs (7) in einen ersten Durchgang (18), der mit dem Holmium-Laserverbindungsstückkanal (13) kommuniziert, und einen zweiten Durchgang (19) teilt, der mit dem Bildbeleuchtungsvorrichtungsverbindungsstückkanal (14) und dem Wassersprühverbindungsstückkanal (9) kommuniziert, wobei die Apertur des zweiten Durchgangs (19) größer als die Apertur des ersten Durchgangs (18) ist, der erste Durchgang (18) koaxial mit dem geraden Holmium-Laserkanalloch (15) kommuniziert, und der zweite Durchgang (19) mit dem geraden Bildbeleuchtungsvorrichtungskanalloch (16) und dem geraden Wassersprühkanalloch (3) kommuniziert.

## Revendications

1. Capuchon à manchon comprenant un collier de logement (1) et un cylindre (2) qui est fixé coaxialement sur une surface intérieure d'une extrémité du collier de logement (1), le cylindre (2) ayant à l'intérieur des trous droits de canal d'instrument et un trou droit de canal de pulvérisation d'eau (3) qui sont parallèles à une direction axiale du cylindre, dans lequel le capuchon à manchon est doté d'un trou oblique de canal de pulvérisation d'eau (4) qui est en communication avec le trou droit de canal de pulvérisation d'eau (3) et peut pulvériser de l'eau vers la face avant latérale du capuchon à manchon ;
**caractérisé en ce que** le trou oblique de canal de pulvérisation d'eau (4) comprend des fentes obliques de canal de pulvérisation d'eau (4.2) chacune étant prévue sur la surface du cylindre (2) et forme un angle avec la direction axiale du cylindre (2), un canal de connexion de pulvérisation d'eau (4.3) est prévu dans le cylindre (2) et est connecté entre l'extrémité arrière de la fente oblique de canal de pulvérisation d'eau (4.2) et le trou droit de canal de pulvérisation d'eau (3), les fentes obliques de canal de pulvérisation d'eau (4.2) sont prévues sur une surface d'un cercle du cylindre (2) dans la direction circonférentielle à intervalles ;
le cylindre (2) est fixé coaxialement à un collier de cylindre (5) qui fait qu'un écoulement d'eau est pulvérisé vers la face avant latérale du capuchon à manchon le long de l'orientation de la fente oblique de canal de pulvérisation d'eau (4.2) et l'extrémité de sortie d'eau de la fente oblique de canal de pulvérisation d'eau (4.2) est située devant le collier de cylindre (5) ; et
le collier de logement (1) et le collier de cylindre (5) sont connectés coaxialement et fixement comme un tout, et le diamètre extérieur du collier de cylindre (5) est le même que le diamètre extérieur du collier de logement (1).

2. Manchon d'introduction d'endoscope avec capuchon à manchon, comprenant une poignée de manchon d'introduction (6) et un tube de manchon d'introduction (7) fixé coaxialement sur l'extrémité avant de la poignée de manchon d'introduction (6), le tube de manchon d'introduction (7) ayant à l'intérieur un canal axial, la poignée de manchon d'introduction (6) étant dotée de jonctions d'instrument et d'une jonction de pulvérisation d'eau (8), les jonctions d'instrument et la jonction de pulvérisation d'eau (8) étant respectivement dotées le long de leurs directions axiales respectives de canaux de jonction d'instrument et d'un canal de jonction de pulvérisation d'eau (9) qui sont en communication avec le canal axial du tube de manchon d'introduction (7), dans lequel le manchon d'introduction d'endoscope comprend en outre le capuchon à manchon selon la revendication 1, une surface intérieure de l'autre extrémité du collier de logement (1) est fixée coaxialement sur l'extrémité avant du tube de manchon d'introduction (7), les trous droits de canal d'instrument et le trou droit de canal de pulvérisation d'eau (3) sont en communication avec le canal axial du tube de manchon d'introduction (7).

3. Manchon d'introduction d'endoscope avec capuchon à manchon selon la revendication 2, dans lequel le tube de manchon d'introduction (7) comprend un tube rigide de manchon d'introduction (7.1) fixé coaxialement sur l'extrémité avant de la poignée de manchon d'introduction (6) et un tube flexible de manchon d'introduction (7.2) fixé coaxialement sur l'extrémité avant du tube rigide de manchon d'introduction (7.1), et l'autre extrémité du collier de logement (1) est connectée coaxialement et fixement sur la surface d'extrémité avant du tube flexible de manchon d'introduction (7.2), le collier de logement (1) est connecté fixement à un câble de traction (9), et le câble de traction (9) passe à travers le canal axial du tube de manchon d'introduction (7) pour être connecté à un dispositif de commande de flexion (10) du tube flexible, et le dispositif de commande de flexion (10) du tube flexible est prévu sur la poignée de manchon d'introduction (6).

4. Manchon d'introduction d'endoscope avec capuchon à manchon selon la revendication 3, dans lequel les jonctions d'instrument comprennent une jonction laser à holmium (11) et une jonction de dispositif d'éclairage d'image (12), la jonction laser à holmium (11) et la jonction de dispositif d'éclairage d'image (12) sont respectivement dotées le long de leurs directions axiales respectives d'un canal de jonction de laser à holmium (13) et d'un canal de jonction de dispositif d'éclairage d'image (14) qui sont en communication avec le canal axial du tube de manchon d'introduction (7), les trous droits du canal d'instrument comprennent un trou droit de canal de laser à holmium (15) et un trou droit de canal de dispositif d'éclairage d'image (16), le trou droit de canal de laser à holmium (15), le trou droit de canal de dispositif d'éclairage d'image (16) et le trou droit de canal de pulvérisation d'eau (3) sont en communication avec le canal axial du tube de manchon d'introduction (7).

5. Manchon d'introduction d'endoscope avec capuchon à manchon selon la revendication 4, dans lequel une cloison axiale (17) est fixée dans le tube de manchon d'introduction (7), et la cloison axiale (17) divise le canal axial du tube de manchon d'introduction (7) en un premier passage (18) en communication avec le canal de jonction de laser à holmium (13) et un second passage (19) en communication avec le canal de jonction de dispositif d'éclairage d'image (14) et le canal de jonction de pulvérisation d'eau (9), l'ouverture du second passage (19) est plus grande que l'ouverture du premier passage (18), le premier passage (18) est en communication coaxiale avec le trou droit de canal de laser à holmium (15), et le second passage (19) est en communication avec le trou droit de canal de dispositif d'éclairage d'image (16) et le trou droit de canal de pulvérisation d'eau (3).
